(19) Europäisches Patentamt
European Patent Office
Office européen des brevets

(11) Publication number: **0 096 116 B1**

(12) **EUROPEAN PATENT SPECIFICATION**

(45) Date of publication of patent specification: **09.04.86**

(51) Int. Cl.$^4$: **C 07 D 333/40,** A 61 K 31/38

(21) Application number: **82112023.5**

(22) Date of filing: **27.12.82**

(54) **Thiophene derivative having a mucoregulating and anticough activity, process for its preparation and pharmaceutical compositions containing it.**

(30) Priority: **21.01.82 IT 1921382**

(43) Date of publication of application:
**21.12.83 *Bulletin 83/51***

(45) Publication of the grant of the patent:
**09.04.86 Bulletin 86/15**

(84) Designated Contracting States:
**AT BE CH DE FR GB IT LI LU NL SE**

(56) References cited:
**FR-A-2 028 682**
**FR-A-2 059 958**

**Chemical Abstracts vol. 94, no. 13, 30 March 1981, Columbus, Ohio, USA R. SCURI et al. "In vivo and in vitro fluidifying activity of sobrerol", page 75, left-hand column, abstract no. 96108f**

(73) Proprietor: **AUSONIA FARMACEUTICI S.r.l.**
**Via Laurentina Km. 24730**
**I-00040 Pomezia (Rome) (IT)**

(72) Inventor: **De Vincentiis, Leonardo**
**42, Isonzo Street**
**Rome (IT)**

(74) Representative: **Bianchetti, Giuseppe**
**Studio Consulenza Brevettuale Via Rossini, 8**
**I-20122 Milan (IT)**

**EP 0 096 116 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

It is an object of the present invention the thiophen-2-carboxylate of 2-methyl-5-(1-hydroxy-1-methyl-ethyl)-cyclohexen-2-yl, having formula I

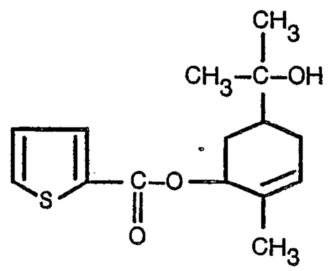

(I)

The therapeutic properties of 2-methyl-5-(1-hydroxy-1-methyl-ethyl)-2-cyclohexen-1-ol, or sobrerol II

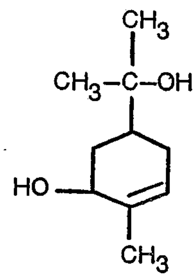

(II)

are known since many years. It has been used for the treatment of respiratory diseases [V. Dalla Valle — Boll. Chim. Farm., 109 (1970)], and more exactly as fluidifying agent with bronchosecretogogue and mucosecretolytic activity in cases of acute and chronic bronchitis with hypersecretive and obstructive component of the respiratory routes [R. Scuri et al., Il Farmaco ed. Pr. *36*, 1 (1980)].

It has now unexpectedly found that the ester of thiophen-2-carboxylic acid with sobrerol, compound I, possesses a bronchosecretogogue, mucosecretolytic and fluidifying activity higher than that of sobrerol and is moreover characterized by marked anti-cough properties, comparable to that of codeine.

Thus, another object of the invention is provided by pharmacological compositions endowed with mucoregulating and anti-cough activities, containing as active principle compound I.

Finally, the invention relates to a process for the preparation of the thiophen-2-carboxylate of 2-methyl-5-(1-hydroxy-1-methyl-ethyl)-cyclohexen-2-yl, characterized by reacting thiophen-2-carboxylic acid chloride III with sobrerol II, according to the following scheme:

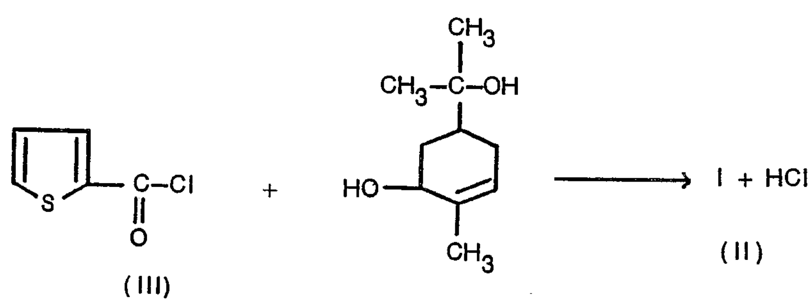


(III) (II)

The reaction is suitably carried out in aprotic solvents, for example in ethers such as diethylether, tetrahydrofuran, dioxane, and in presence of acidity acceptors. As such, tertiary bases are preferably used, such as pyridine or triethylamine. One can operate at temperatures ranging from 0 and 40°C, preferably at room temperature.

The following example further illustrates without limiting it, the process according to the invention.

Example

60 Grams of thiophen-2-carboxylic acid chloride are dissolved in 500 ml of anhydrous tetrahydrofuran; to this solution 30 ml of anhydrous pyridine and 60 g of sobrerol (in its dl-trans form), diluted in 500 ml of anhydrous tetrahydrofuran, are added. The mixture is allowed to react for two hours at room temperature. At the end, the solvent is evaporated under reduced pressure; the residue is diluted by ethylether and the organic phase is washed first with water, then with a saturated bicarbonate solution. The organic phase is dried over anhydrous sodium sulphate, filtered and the solvent is evaporated under reduced pressure. The

crude product so obtained is purified on a silica gel chromatographic column by eluting first with petroleum ether and then with a mixture of petroleum ether/ethyl ether 1:1.

73 Grams of an oil, impossible to crystallize but perfectly pure in TLC, are obtained. The compound so obtained, of a yellowish colour, is almost insoluble in water, and soluble in the common organic solvents.

Elemental analysis:
for $C_{15}H_{20}O_3S$ (M.W. = 280.374)
calcd.% C = 64.26; H = 7.19; S = 11.43
found% C = 64.09; H = 7.24; s = 11.38.
The structure of compound I is confirmed by spectroscopical data.

IR Spectrum (liquid film; values of absorption bands are expressed in $cm^{-1}$):

| | |
|---|---|
| stretch O − H broad band | 3560—3300 |
| stretch C = O | 1710 |
| stretch C − O | 1050 |

$H^1$ NMR Spectrum (recorded in $CDCl_3$; internal reference TMS; values of chemical shifts are expressed in δ):

$CH_3$
|
1.2 (d, 6H, —C—$CH_3$);

1.3—2.5 (m, 9H, =C—$CH_3$, $\underline{CH_2}$—$\underline{CH}$—$\underline{CH_2}$, OH mobile);
5.5 (m, 1H, C$\underline{H}$—O—CO);
5.8 (m, 1H, C=CH—);
7.1 (double t, 1H in $C_4$ of thiophen ring);
7.5 (double d, 1H in $C_5$ of thiophen ring);
7.8 (double d, 1H in $C_3$ of thiophen ring).

The bio-pharmacological characteristics of compound I have been determined as hereinunder described.

Toxicity

The acute toxicity has been determined in Swiss mouses and in Wistar rats for different administration routes, according to the method of Litchfield J. T. and Wilcoxon [J. Pharm. Exp. Therap. *96*, 99—113 (1949)].

The results obtained are reported in Table 1; from the same it can be pointed out how I is endowed with a very low acute toxicity.

TABLE 1
Acute toxicity of I

| Species | Administration route | $LD_{50}$ — (mg/kg) |
|---|---|---|
| mouse | os | >4000 |
| | i.p. | 2500 |
| rat | os | >4000 |
| | i.p. | 2650 |

Broncosecretogogue activity

The broncosecretogogue activity has been determined in male Wistar rats by per os administration according to the method of Mawatari [Mawatari H., Kagoshima Daigaku, Igaku-Zasshi, *27*, 561 (1976)], comparing I with carboxymethylcysteine, sobrerol and guaiphenesin at the doses hereinafter stated. The results are reported in the Table 2.

TABLE 2
Bronchosecretogogue activity (Mawatari technique)

| Compound | Administered Dose mg/kg/os | N° of animals | % Increase of FlNa(*) in comparison with controls |
|---|---|---|---|
| I | 500 | 10 | 76.4 |
| Carboxymethyl-cysteine | 500 | 10 | 39.7 |
| Sobrerol | 500 | 10 | 33.4 |

(*) FlNa = Sodium fluoresceine.

From the resulting values a more marked activity of I in the test under exam clearly turns out in comparison with reference drugs used at equiponderant doses.

Mucosecretolytic activity

The mucosecretolytic activity has been studied in male rabbits according to the technique of Giraldez, Grass and Bruseghini (Abstr. Comp. Int. Pharm. N. 1086-Paris 1978), by administration by the oral route, comparing I with carboxymethylcysteine and sobrerol at the dose hereinafter stated. The results are reported in Table 3.

TABLE 3
Mucosecretolytic activity (technique) of Giraldez et al.)

| Compound | Dose mg/kg/os | N° animals | % Increase of secretion in comparison with controls |
|---|---|---|---|
| I | 500 | 10 | 54.1 |
| Carboxymethyl-cysteine | 500 | 10 | 40.2 |
| Sobrerol | 500 | 10 | 36.8 |

From the comparison of the results obtained by the test under exam the mucosecretolytic activity of I results comparable to that of carboxymethylcysteine and sobrerol used at equiponderant doses.

Anti-cough activity

The anti-cough activity of I has been assessed after administration by endoperitoneal route (30% in tween) on rabbits of both sexes, weighing 200—350 g, according to the method of the cough induced by citric acid aerosol administration, and evaluating the mean number of cough strokes in comparison with the same basal values (Boura and coll. — Prog. Brit. Pharmac. Soc. Apr. 1970). As reference standard a group of codeine treated animals at the dose of 12.5 mg/kg has been used.

TABLE 4
Anti-cough activity

| Treatment | Dose mg/kg | N° animals | % Inhibition in comparison with controls | Statistical significance (basal minus final values) |
|---|---|---|---|---|
| Controls | — | 12 | 26.7 | — |
| Codeine | 12.5 | 12 | 76.7 | $p < 0.05$ |
| I | 250 | 12 | 78.6 | $p < 0.05$ |

From the above stated results it becomes evident the very good anti-cough activity of I in comparison with traditional standard of proved activity, in the used experimental conditions.

Fluidifying activity "in vivo"

The fluidifying effect has been determined with the microviscosimeter on the tracheo-bronchial mucus from rabbits after administration by oral route, comparing I with sobrerol, at the doses hereinafter stated. The results are reported in Table 5.

TABLE 5
Fluidifying activity "in vivo" in the rabbit

| Compound | Dose mg/kg/os | N° of animals | % Reduction of the mucus viscosity |
|---|---|---|---|
| I | 500 | 5 | 39.8 |
| Sobrerol | 500 | 5 | 15.6 |

As it turns out from the values obtained, the fluidifying activity of I proves to be remarkably enhanced in comparison with the reference drug employed at equiponderant dose.

Pharmacokinetics

From the pharmacokinetic tests performed in the animal after oral administration of I and sobrerol in equimolar doses, higher sobrerol concentrations at pulmonary level have been surprisingly noticed, as a demonstration of a higher pulmonary tropism for I.

Compound I is therefore fit as an elective drug with mucoregulating, mucolytic and anti-cough activities, in the therapy of acute and chronic diseases of the respiratory apparatus, particularly of acute and chronic bronchitis and of cough of any nature.

The present invention refers also to all the industrially applicable aspects connected with the use of I as mucoregulating and anti-cough agent. An essential aspect of the invention is therefore provided by pharmaceutical compositions containing predetermined amounts of I. Such a composition can be administered, particularly, by the oral route, as capsules, extemporary suspension, monodose sachets (in a granular state), or by aerosol; or as suppositories; or, finally, by parenteral route (vials for injection). Of course, the single pharmaceutical forms will contain the active principle together with vehicles, excipients, flavouring agents etc. of conventional use in pharmaceutical technique.

**Claims for the Contracting States: BE CH DE FR GB IT LI LU NL SE**

1. Thiophen-2-carboxylate of 2-methyl-5-(1-hydroxy-1-methyl-ethyl)-cyclohexen-2-yl, having formula I

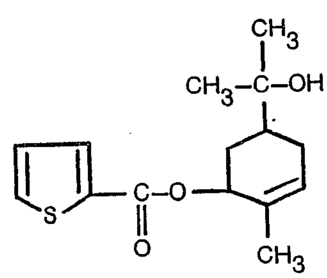

(I)

2. Pharmaceutical composition endowed with bronchosecretogogue, mucosecretolytic and anti-cough activities, characterized by containing as active principle the compound of claim 1.

3. Process for the preparation of the thiophen-2-carboxylate of 2-methyl-5-(1-hydroxy-1-methyl-ethyl)-cyclohexen-2-yl, having formula I, characterized by reacting thiophen-2-carboxylic acid chloride III with sobrerol II, according to the following scheme:

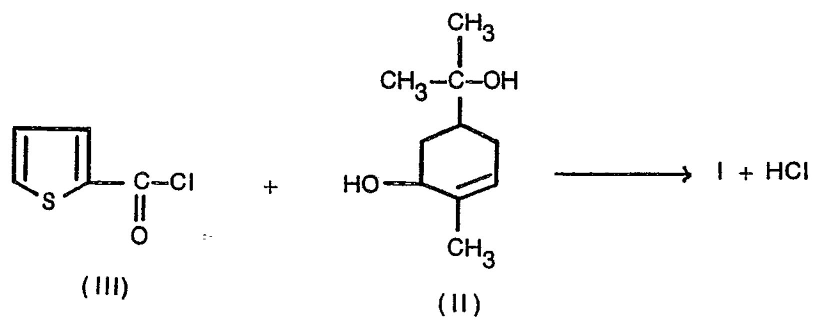

4. Process according to claim 3, characterized by operating in aprotic solvents.

5. Process according to claims 3—4, characterized by operating in the presence of acidity acceptors.

6. Process according to claims 3—5, characterized by operating at temperatures ranging from about 0 to 40°C.

**Claims for the Contracting State: AT**

1. Process for the preparation of the thiophen-2-carboxylate of 2-methyl-5-(1-hydroxy-1-methyl-ethyl)-cyclohexen-2-yl, having formula I

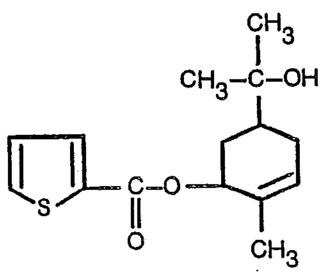

(I)

characterized by reacting thiophen-2-carboxylic acid chloride III with sobrerol II, according to the following scheme:

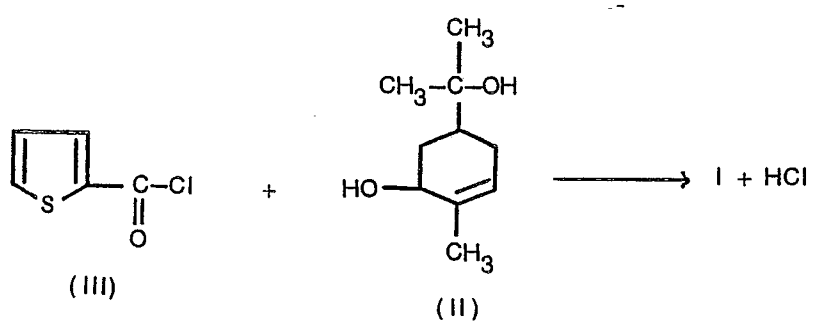

2. Process according to claim 1, characterized by operating in aprotic solvents.

3. Process according to claims 1—2, characterized by operating in the presence of acidity acceptors.

4. Process according to claims 1—3, characterized by operating at temperatures ranging from about 0 to 40°C.

**Patentansprüche für die Vertragsstaaten: BE CH DE FR GB IT LI LU NL SE**

1. 2-Methyl-5-(1-hydroxy-1-methyl-äthyl)-cyclohexen-2-yl-thiophen-2-carboxylat der Formel I

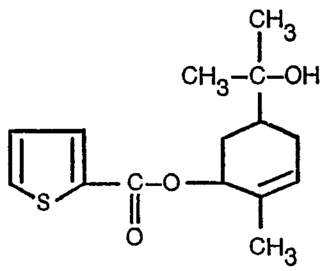

(I)

2. Arzneimittel, ausgestattet mit bronchialsekretionsanregender, mucosekretolytischer und Anti-Husten-Wirkung, dadurch gekennzeichnet, daß es als Wirkstoff die Verbindung nach Anspruch 1 enthält.

3. Verfahren zur Herstellung des 2-Methyl-5-(1-hydroxy-1-methyl-äthyl)-cyclohexen-2-yl-thiophen-2-carboxylats der Formel I, dadurch gekennzeichnet, daß man Thiophen-2-carbonsäurechlorid III mit Sobrerol II gemäß dem folgenden Schema umsetzt:

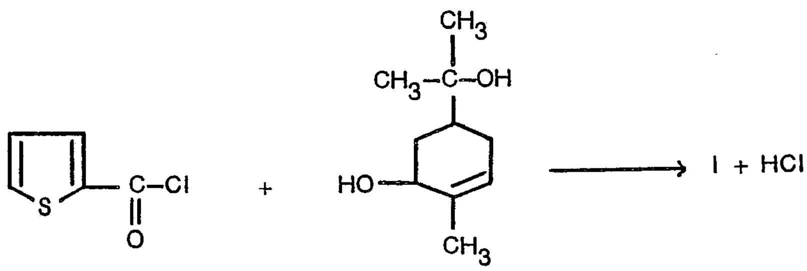


(III) (II)

4. Verfahren nach Anspruch 3, dadurch gekennzeichnet, daß man in aprotischen Lösungsmitteln arbeitet.

5. Verfahren nach den Ansprüchen 3 bis 4, dadurch gekennzeichnet, daß man in Gegenwart von Säureacceptoren arbeitet.

6. Verfahren nach den Ansprüchen 3 bis 5, dadurch gekennzeichnet, daß man bei Temperaturen im Bereich von 0 bis 40°C arbeitet.

**Patentansprüche für den Vertragsstaat: AT**

1. Verfahren zur Herstellung des 2-Methyl-5-(1-hydroxy-1-methyl-äthyl)-cyclohexen-2-yl-thiophen-2-carboxylats der Formel I

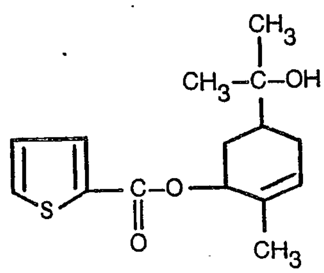

(I)

dadurch gekennzeichnet, daß man Thiophen-2-carbonsäurechlorid III mit Sobrerol II gemäß dem folgenden Schema umsetzt:

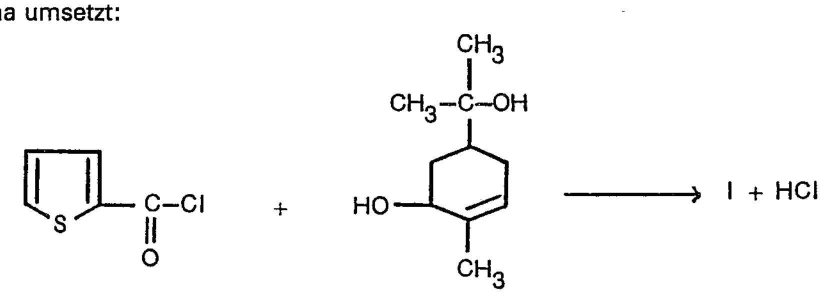


(III) (II)

2. Verfahren nach Anspruch 1, dadurch gekennzeichnet, daß man in aprotischen Lösungsmitteln arbeitet.

3. Verfahren nach den Ansprüchen 1 bis 2, dadurch gekennzeichnet, daß man in Gegenwart von Säureacceptoren arbeitet.

4. Verfahren nach den Ansprüchen 1 bis 3, dadurch gekennzeichnet, daß man bei Temperaturen im Bereich von 0 bis 40°C arbeitet.

**Revendications pour les Etats contractants: BE CH DE FR GB IT LI LU NL SE**

1. Le thiophen-2-carboxylate de 2-méthyl-5-(1-hydroxy-1-méthyl-éthyl)-cyclohexen-2-yle, répondant à la formule I

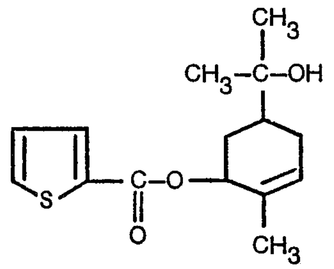

(I)

2. Composition pharmaceutique dotée d'activités bronchosécrétagogue, mucosécrétolytique et antitussive, caractérisée en ce qu'elle contient à titre de principe actif le composé selon la revendication 1.

3. Procédé de préparation du thiophen-2-carboxylate de 2-méthyl-5-(1-hydroxy-1-méthyl-éthyl)-cyclohexen-2-yle, répondant à la formule I, caractérisé en ce qu'il consiste à faire réagir le chlorure d'acide thiophen-2-carboxylique III avec le sobrérol II selon le schéma de réaction suivant:

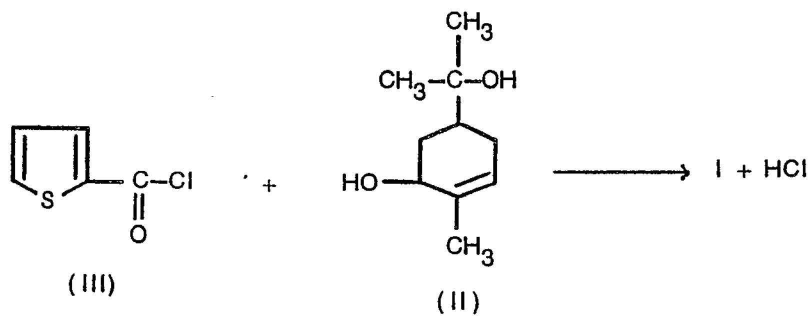


(III) (II)

4. Procédé selon la revendication 3, caractérisé en ce que la réaction est effectuée dans des solvants aprotiques.

5. Procédé selon les revendications 3—4, caractérisé en ce que la réaction est effectuée en présence d'accepteurs d'acides.

6. Procédé selon les revendications 3—5, caractérisé en ce que la réaction est effectuée à des températures comprises dans une plage allant de 0 à 40°C.

**Revendications pour l'Etat contractant: AT**

1. Procédé de préparation du thiophen-2-carboxylate de 2-méthyl-5-(1-hydroxy-1-méthyl-éthyl)-cyclohexen-2-yle, répondant à la formule I:

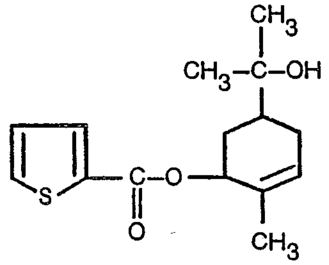

(I)

caractérisé en ce qu'il consiste à faire réagir le chlorure d'acide thiophen-2-carboxylique III avec le sobrérol II selon le schéma de réaction suivant:

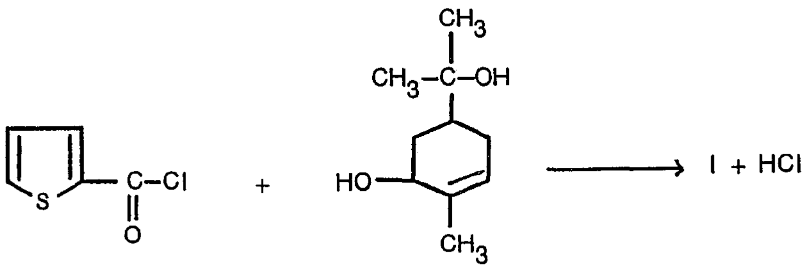


(III) (II)

2. Procédé selon la revendication 1, caractérisé en ce que la réaction est effectuée dans des solvants aprotiques.

3. Procédé selon les revendications 1—2, caractérisé en ce que la réaction est effectuée en présence d'accepteurs d'acides.

4. Procédé selon les revendications 1—3, caractérisé en ce que la réaction est effectuée à des températures comprises dans une plage allant de 0 à 40°C.